# FASCICULE DE BREVET EUROPEEN

(11) **EP 1 648 482 B1**
(45) Date de publication et mention de la délivrance du brevet: **20.03.2013**
(21) Numéro de dépôt: 04767729.9
(22) Date de dépôt: 19.07.2004
(51) Int. Cl.: A61K 36/48, A61Q 11/00, A61Q 19/00, A61Q 19/08, A61K 8/97, A61K 31/56

(54) **UTILISATION D'UNE COMPOSITION COSMETIQUE OU PHARMACEUTIQUE COMPRENANT UN EXTRAIT RICHE EN LUPEOL A TITRE DE PRINCIPE ACTIF POUR STIMULER LA SYNTHESE DES PROTEINES DE STRESS**
VERWENDUNG EINER KOSMETISCHEN ODER PHARMAZEUTISCHEN ZUSAMMENSETZUNG MIT EINEM LUPEOLREICHEN EXTRAKT ALS WIRKSTOFF ZUR STIMULIERUNG DER SYNTHESE VON HITZESCHOCKPROTEINEN
USE OF A COSMETIC OR PHARMACEUTICAL COMPOSITION, COMPRISING A LUPEOL-RICH EXTRACT AS AN ACTIVE INGREDIENT FOR STIMULATING THE SYNTHESIS OF HEAT SHOCK PROTEINS

(30) Priorité: 18.07.2003 FR 0308796
(43) Date de publication de la demande: 26.04.2006
(73) Titulaire: Laboratoires Expanscience, 92400 Courbevoie (FR)
(72) Inventeur: MSIKA, Philippe, F-78000 Versailles (FR); PICCIRILLI, Antoine, F-78000 Versailles (FR); PICCARDI, Nathalie, F-38120 Saint Egreve (FR)
(74) Mandataire: Regimbeau
(86) Numéro de dépôt international: PCT/FR2004/001907
(87) Numéro de publication internationale: WO 2005/009331

(56) Documents cités:
- WO-A-98/47479
- FR-A- 2 778 565
- GOGLY B ET AL: "Effects of vegetable extract from lupinus albus (Lu105) on the degradation by human leucocyte elastase and bacterial collagenase of gingival collagen and elastic fibers" JOURNAL OF INVESTIGATIVE DERMATOLOGY, vol. 119, no. 1, juillet 2002 (2002-07), page 232, XP002286368 & 63RD ANNUAL MEETING OF THE SOCIETY FOR INVESTIGATIVE DERMATOLOGY; LOS ANGELES, CALIFORNIA, USA; MAY 15-18, 2002 ISSN: 0022-202X
- SALEEM MOHAMMAD ET AL: "Lupeol, a triterpene, inhibits early responses of tumor promotion induced by benzoyl peroxide in murine skin" PHARMACOLOGICAL RESEARCH, vol. 43, no. 2, février 2001 (2001-02), pages 127-134, XP002286369 ISSN: 1043-6618

## Description

La présente invention concerne l'utilisation d'un extrait riche en lupéol pour la fabrication d'une composition pharmaceutique ou cosmétique destinée à traiter et/ou prévenir une dégénérescence des tissus conjonctifs.

Le lupin est une plante assez répandue, que l'on trouve en Europe, en Asie, ainsi qu'en Amérique du Nord et du Sud. Ce végétal est un proche parent du pois, de la fève, du soja et du haricot. Plusieurs espèces de lupin peuvent être citées comme étant les plus connues : lupinus albus (lupin blanc), lupinus angustifolius (lupin bleu), lupinus luteus (lupin jaune), lupinus mutabilis (lupin changeant), lupinus graecus, lupinus micranthus Guss, lupinus hispanicus, lupinus pilosus, lupinus cosentinii, lupinus atlanticus, lupinus princei et lupinus somaliensis. Une des espèces les plus courantes sur le territoire européen est le lupin blanc doux (lupinus albus), notamment la variété Arès présentant le gène pauper.
Le lupéol (1) appartient à la famille des triterpènes et plus particulièrement à celle des alcools triterpéniques.

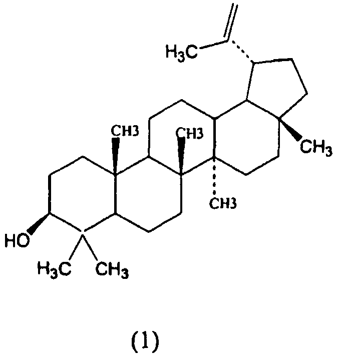

Le lupéol présente un intérêt certain de par ses nombreuses activités biologiques. Il est notamment connu pour ses propriétés anti-inflammatoires (Singh S. et coll., Filoterapia, 1997, 68, No. 1, 9) et analgésiques (De Miranda A. L. et coll., Planta Med, 2000, 66(3), 284), son action nephroprotectrice vis-à-vis des métaux lourds (Nagaraj M. et coll., J. Appl. Toxicol, 2000, 20(5), 413), son action anti-histaminique (De Medrano Villar M. J. et coll., Methods Find Exp. Clin. Pharmacol., 1997, 19, No. 8, 515), ses activités anti-mitotiques (Zachariah R. et coll., Indian J. Pharm. Sci., 1994, 56, No. 4, 129) et anti-virales (Kahlos K., *Filoterapia,* 1996, 67, No. 4, 344).

Le lupéol est aussi connu pour ses propriétés anti-oxydantes. En effet, le lupéol active la régénération des enzymes anti-oxydantes cutanées, qui ont été endommagées par les toxines environnementales. De plus, il est un agent chimiopréventif de la peau et il permet la suppression de la toxicité cutanée induite par le péroxyde de benzoyle (Saleem M, et al., 2001, Lupeol, a triterpene, inhibits early responses of tumor prévention induced by benzoyl peroxide in murine skin, Pharmacol Res, 43(2) :127-134).

Le lupéol permet le contrôle de la prolifération des kératinocytes, et il peut donc être utilisé dans des compositions anti-inflammatoires.

Il a également été trouvé que le lupéol permet de réduire efficacement les risques de formation de calculs et il est donc utilisé dans le traitement des maladies urinaires (Malini MM, et al. (2000) Protective effect of triterpenes on calcium oxalate crystal induced peroxidative changes in experimental urolithiasis. Pharmacol Res. 41(4) :413-418).

Le lupéol peut également être utilisé en tant qu'intermédiaire de synthèse, notamment pour la préparation de phyto-hormones et d'analogues de stéroides.

Le lupéol est présent dans de nombreux végétaux tels que l'Aloe vera ou l'écorce de Crataeva nurvala. Il a été isolé à plusieurs reprises à partir de plantes diverses comme le Bresk.

Il a également été extrait des coques de lupin. La demande de brevet FR 2 822 821 décrit un extrait de coques de graines de lupin contenant du lupéol, avantageusement, l'extrait présente un taux de lupéol supérieur à 30% en poids, de préférence supérieur à 50% en poids. De manière encore plus avantageuse, l'extrait de coques de graines de lupin présente un taux de lupéol compris entre 70 et 100%.

La demande de brevet FR 2 822 821 décrit également un procédé d'obtention d'un extrait de coques de graines de lupin, qui comprend au moins la succession d'étapes suivantes :
- broyage des coques de lupin,
- extraction des lipides totaux contenus dans les coques de lupin broyées à l'aide d'un solvant organique choisi dans le groupe constitué par les alcanes aliphatiques, les alcanes aromatiques, les alcools aliphatiques et leurs dérivés halogénés, et
- purification des lipides obtenus pour obtenir un extrait riche en lupéol.

Les protéines du choc thermique (famille des HSP, Heat stress ou shock protein), ci après dénommées protéines de stress et abrégées HSP, sont présentent dans tous les organismes des bactéries jusqu'à l'homme. Il existe 5 groupes majeurs de HSP caractérisés par leur poids moléculaire :
■ 20-30 kDa ;
■ 50-60 kDa ;
■ 70 kDa ;
■ 90 kDa ; et
■ 100-110 kDa.

L'ubiquitine, ainsi que l'hème oxygénase appartiennent également à la grande famille des HSPs.

Les HSP sont exprimées de manière constitutionnelle dans des conditions physiologiques et possèdent des activités basiques et indispensables à la synthèse protéique, tout en protégeant les cellules de nombreuses agressions telle qu'une élévation de température, le rayonnement ultra-violet, l'ischémie-reperfusion et l'apopotose.

La fonction chaperon des HSP est l'une des grandes fonctions protéiques à avoir été découverte. Initialement mise en évidence dans le cadre d'un stress thermique, la présence de ces protéines à un niveau déjà important dans toutes cellules en l'absence de stress a suggéré qu'elles participaient au repliement normal des protéines. Ces chaperones sont capables d'inhiber l'agrégation de protéines partiellement dénaturées et de les replier en utilisant de l'ATP. Elles empêchent les associations inappropriées de protéines, et sont impliquées dans le transport intracellulaire et la sécrétion des protéines.

L'HSP47 est une protéine localisée dans le réticulum endoplasmique des cellules (compartiment de la synthèse protéique) et elle se fixe spécifiquement sur différents types de collagène et de pro-collagène, notamment de type I, II, III, IV et V. L'HSP47 est impliquée dans le « système contrôle qualité » de la cellule, empêchant que cette dernière ne sécrète du pro-collagène avec une structure inadéquate. Une corrélation entre sur-expression du collagène et de l'HSP47 a été démontrée chez l'homme.

Les collagènes représentent environ 30% des protéines totales et sont les constituants majeurs de la matrice extracellulaire des tissus conjonctifs tels que le derme (70% des protéines de la matrice extracellulaire), le cartilage ou le tissu conjonctif gingival (60 à 65% des protéines). A ce jour, 19 types de collagène différents ont été décrits. Par définition, ils possèdent des domaines en triple hélice en proportions variables et forment des structures organisées au sein de la matrice extracellulaire comme des fibrilles, des filaments ou des réseaux. Les collagènes sont synthétisés à partir d'un pro-collagène par les cellules conjonctives telles que les fibroblastes dermiques et gingivaux, ainsi que par les chondrocytes au niveau du cartilage articulaire. Les fibres de collagène synthétisées se renforcent et s'assemblent en faisceaux qui constituent la fibre collagène définitive et insoluble, qui pourra répondre dans l'avenir aux contraintes de traction.

Les tissus conjonctifs jouent un rôle majeur en tant que support et soutien pour les autres éléments constitutifs de l'organe et en tant qu'absorbeur de choc. Par exemple le tissu conjonctif gingival assure le soutien des dents. Au niveau cutané, le derme est notamment responsable de la fermeté de la peau.

Une dégénérescence des tissus conjonctifs, liée à une altération du réseau collagénique, telle qu'une inhibition de la synthèse du collagène et/ou du pro-collagène, une synthèse imparfaite du collagène et/ou du pro-collagène, une dégradation des fibres de collagène, une diminution du nombre des fibroblastes et de leur métabolisme, peuvent donc avoir des conséquences importantes.

La Demanderesse a découvert qu'un extrait riche en lupéol peut également être utilisé pour stimuler la synthèse des protéines de stress et plus particulièrement pour stimuler la synthèse de l'HSP47 (Heat Schock Protein 47) afin de traiter et/ou de prévenir la dégénérescence des tissus conjonctifs relative à des processus internes et/ou à des agressions extérieures.

La Demanderesse a ainsi découvert qu'un extrait riche en lupéol peut permettre de contrôler la structure normale du collagène, via la stimulation de à synthèse de l'HSP47, lorsque la peau et/ou les articulations et/ou les tissus conjonctifs gingivaux sont soumis à un environnement stressant. Cet environnement stressant peut être une contrainte physique, induite par exemple par une prise de poids pouvant notamment entraîner une variation du phénotype et du métabolisme des fibroblastes, une expositions aux radiations, notamment aux radiations UV, une sénescence ou une inflammation.

Par le terme d'extrait "riche en lupéol", on entend au sens de la présente invention, un extrait présentant un taux de lupéol supérieur à 30% en poids, avantageusement supérieur à 50% en poids, et de manière encore plus avantageuse compris entre 70 et 100% en poids.

La présente invention a pour principal objet l'utilisation d'un extrait riche en lupéol pour la fabrication d'une composition cosmétique ou pharmaceutique destinée à traiter et/ou prévenir la dégénérescence des tissus conjonctifs au niveau du derme et/ou du cartilage, et/ou du tissu conjonctif gingival, liée à une anomalie de la production du collagène.

Un extrait riche en lupéol selon la présente invention peut ainsi être utilisé dans une composition cosmétique destinée à traiter et/ou prévenir la dégénérescence des tissus conjonctifs au niveau du derme et/ou du cartilage, et/ou du tissu conjonctif gingival. Un extrait riche en lupéol peut également être utilisé pour la fabrication d'un médicament destiné à traiter et/ou prévenir la dégénérescence des tissus conjonctifs au niveau du derme et/ou du cartilage, et/ou du tissu conjonctif gingival.

Dans le cadre de la présente invention, on parlera indifféremment de composition pharmaceutique ou de médicament.

Au sens de la présente invention, on entend par « anomalie de la production du collagène », une synthèse insuffisante du collagène et/ou du pro-collagène par les fibroblastes ou les chondrocytes, une synthèse imparfaite des collagènes et/ou des pro-collagènes, une dégradation des fibres de collagènes, une diminution du nombre de fibroblastes ou de chondrocytes et de leur métabolisme.

Au sens de la présente invention, on entend par « synthèse imparfaite » du collagène, toute synthèse d'un collagène ou pro-collagène présentant des anomalies de structure, ne pouvant plus notamment remplir sa fonction de soutien mécanique de la peau et/ou des articulations et/ou des tissus conjonctifs gingivaux.

L'anomalie de la production du collagène peut être induite par un facteur stress interne ou externe. Par exemple, sous une contrainte physique, telle qu'une prise de poids, une surcharge pondérale, une distension des tissus notamment dans le cadre d'une grossesse, à la suite d'une irradiation, notamment aux rayons ultraviolets, suite à une inflammation ou dans un état de sénescence, les fibroblastes ou les chondrocytes, soumis à ces contraintes externes, peuvent synthétiser du collagène de structure anormale.

L'anomalie de la production du collagène peut notamment être une inhibition de la synthèse ou une synthèse imparfaite des pro-collagènes et collagènes, en particulier des pro-collagènes et/ou collagènes du type I, II, III, IV ou V.

L'extrait riche en lupéol selon la présente invention joue le rôle d'un promoteur de la synthèse des protéines de stress, notamment de la synthèse de l'HSP47. Cette activation des protéines de stress, avantageusement de l'HSP47, permet de stimuler le métabolisme et la prolifération des fibroblastes et des chondrocytes et ainsi de lutter contre et/ou de prévenir la dégénérescence du réseau collagénique.

La composition cosmétique selon l'invention est destinée à stimuler la synthèse des protéines de stress HSP, en particulier la synthèse de l'HSP47, et ainsi l'activité des fibroblastes et des chondrocytes.

La composition pharmaceutique ou le médicament selon l'invention est destiné à stimuler la synthèse des protéines de stress HSP, en particulier la synthèse de l'HSP47, et ainsi l'activité des fibroblastes et des chondrocytes.

Selon une variante avantageuse de l'invention, la composition pharmaceutique ou le médicament est destiné à traiter et/ou prévenir les pathologies articulaires, notamment les pathologies articulaires non inflammatoires.

Les pathologies articulaires sont caractérisées par un déséquilibre entre la synthèse et la dégradation de la matrice extracellulaire qui entoure les chondrocytes. Le cartilage articulaire, qui est principalement constitué d'un réseau de fibres de collagène, d'un gel de protéoglycanes hydrophiles et de chondrocytes, joue un double rôle mécanique essentiel pour la protection de l'os sous-chondral. D'une part il diminue les forces de frottement lors du déplacement des segments osseux et, d'autre part, il assure la transmission, la répartition et l'amortissement des contraintes subies par l'articulation. A l'âge adulte, le renouvellement du réseau collagénique au sein du cartilage articulaire est très faible. Il est donc important de pouvoir stimuler la synthèse des collagènes et des pro-collagènes et/ou atténuer une altération du réseau collagénique, qui sont les constituants majeurs du cartilage. La composition pharmaceutique selon la présente invention permet la stimulation de la synthèse de l'HSP47 qui permet ainsi de lutter contre et/ou de prévenir la dégénérescence du tissu conjonctif du cartilage articulaire.

Avantageusement, le médicament selon l'invention est destiné à prévenir et/ou traiter l'arthrose, qui peut être due à une lésion primitive du cartilage avec modification de la structure des tissus conjonctifs et une diminution des protéoglycannes.

Le médicament ou la composition pharmaceutique selon l'invention est également destiné à prévenir et/ou traiter les maladies parodontales. Particulièrement, la composition pharmaceutique selon l'invention est destinée à prévenir et/ou traiter la gingivite ou la parodontite.

Les maladies parodontales, dont la conséquence ultime est la perte de la dent, se traduisent notamment par une dégénérescence des tissus conjonctifs gingivaux. Le tissu conjonctif gingival est composé de 60 à 65 % de collagènes. La composition pharmaceutique selon la présente invention permet de lutter et/ou de prévenir la dégénérescence du tissu conjonctif gingival, via une stimulation de la synthèse de l'HSP47 qui va permettre de promouvoir l'expression du collagène.

Selon une autre variante avantageuse de l'invention, le médicament selon l'invention est destiné à la prévention et/ou au traitement des vergetures.

Les vergetures peuvent être considérées comme une atteinte de la cellule fibroblastique caractérisée notamment par une inhibition de l'expression des gènes codants pour la fibronectine, les collagènes de type I et II et une transformation des fibroblastes en myofibroblastes sous l'effet des distensions mécaniques. Cette dégénérescence du tissu collagénique conduit à la formation d'une cicatrice dermique atrophique. Un des principaux facteurs déclenchant est le stress mécanique.

La composition pharmaceutique selon la présente invention est avantageusement à application topique. On entend par application topique aussi bien une application de la composition au niveau de la peau qu'au niveau buccal, notamment au niveau des gencives.

La composition pharmaceutique selon l'invention se caractérise en ce que la concentration en extrait riche en lupéol est comprise entre 0,001% et 10 % en poids, avantageusement entre 0,1 et 10 % en poids, en particulier entre 1 et 5 % en poids, par rapport au poids total de la composition pharmaceutique.

La composition cosmétique selon la présente invention permet de favoriser la résistance au stress et aux contraintes mécaniques de la peau et/ou des articulations et/ou des tissus conjonctifs gingivaux.

Ladite composition cosmétique permet aux tissus conjonctifs de résister au stress sous une contrainte physique, telle qu'une prise de poids, une surcharge pondérale, une distension des tissus notamment dans le cadre d'une grossesse, une irradiation, notamment aux ultra-violets.

Selon une variante avantageuse de l'invention, la composition cosmétique est utile en tant qu'agent cicatrisant de la peau et/ou des articulations et/ou des tissus conjonctifs gingivaux et/ou des muqueuses. En effet, la composition cosmétique selon l'invention permet de stimuler l'activité des fibroblastes et des chondrocytes, qui synthétisent le collagène nécessaire pour combler la perte de substance par un nouveau tissu.

La composition cosmétique est avantageusement utilisée pour notamment prévenir et/ou traiter les vergetures ou leur apparition.

Selon une autre variante avantageuse de l'invention, la composition cosmétique est utile en tant qu'agent restructurant de la peau et/ou des muqueuses.

La composition cosmétique est également avantageusement utile en tant qu'agent anti-relâchement de la peau et/ou des muqueuses. Le viellissement cutané est notamment caractérisé par une diminution de nombre de fibroblastes ainsi que par une diminution de leur activité. La composition cosmétique selon l'invention permet de stimuler le métabolisme et la prolifération des fibroblastes. Elle est donc avantageusement utilisée pour prévenir et/ou retarder le vieillissement cutané chronologique, extrinsèque, notamment dû au soleil, au tabac, à la pollution, au stress, et ménopausique.

La composition cosmétique selon l'invention agit au niveau du derme de la peau. Selon une variante avantageuse de l'invention, la composition cosmétique est destinée aux femmes enceintes.

La composition cosmétique peut aussi être utilisée en tant que bain de bouche ou pâte dentifrice.

Au sens de la présente invention, l'expression « pâte dentifrice» inclut toutes les formulations classiques de dentifrice, que ce soit notamment sous la forme d'un gel de dentifrice, d'une pâte de dentifrice ou d'une combinaison pâte et gel de dentifrice.

La composition cosmétique est avantageusement à application topique. On entend par application topique aussi bien une application de la composition au niveau de la peau qu'au niveau buccal, notamment au niveau des gencives.

La composition cosmétique selon l'invention se caractérise en ce que la concentration en extrait riche en lupéol est comprise entre 0,001 et 10% en poids, avantageusement entre 0,1 et 10 % en poids, en particulier entre 1 et 5 % en poids, par rapport au poids total de la composition cosmétique.

L'extrait riche en lupéol présente un taux de lupéol supérieur à 30% en poids, avantageusement supérieur à 50% en poids, et de manière encore plus avantageuse compris entre 70 et 100% en poids.

Ainsi, la composition cosmétique selon l'invention se caractérise en ce que la concentration en lupéol est comprise entre 0,0003 et 10% en poids, avantageusement entre 0,007 et 10% en poids, par rapport au poids total de la composition cosmétique.

La composition qui permet la mise en oeuvre de l'invention comprend un support cosmétiquement acceptable, c'est à dire un support compatible avec la peau et peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et ou non-ionique, d'un dispositif trans-dermique ou sous toute autre forme pour application topique.

Cette composition peut être plus ou moins fluide et avoir l'aspect d'une crème blanche ou colorée, d'une pommade, d'un lait, d'une lotion, d'un sérum, d'une pâte, d'une mousse ou d'un gel.

Elle peut éventuellement être appliquée sur la peau sous forme d'aérosol. Elle peut également se présenter sous forme solide, et par exemple sous forme de stick. Elle peut aussi être appliquée au moyen d'un patch.

Avantageusement, le milieu cosmétiquement acceptable est une solution huileuse, une émulsion eau-dans-huile, une émulsion huile-dans eau, une microémulsion, un gel huileux, un gel anhydre, une dispersion de vésicules, de microcapsules ou de microparticules, un dispositif trans-dermique.

La composition selon l'invention peut contenir également les adjuvants habituels dans le domaine cosmétique, tels que les gélifiants hydrophiles ou lipophiles, les actifs hydrophiles ou lipophiles, les épaississants, les conservateurs, les antioxydants, les solvants, les parfums, les agents chélateurs, les absorbeurs d'odeur, des filtres chimiques ou minéraux, des pigments minéraux, les tensioactifs, les polymères, les huiles de silicone et les matières colorantes. Les quantités de ces différents adjuvants sont celles classiquement utilisées dans les domaines considérés, et par exemple de 0,01 à 20% du poids total de la composition. Ces adjuvants, selon leur nature, peuvent être introduits dans la phase grasse, dans la phase aqueuse, dans les vésicules lipidiques et ou dans les nanoparticules.

Lorsque la composition de l'invention est une émulsion, la proportion de la phase grasse peut aller de 5 à 80% en poids, et de préférence de 5 à 50% du poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30% en poids, et de préférence de 0,5 à 20% du poids total de la composition.

Comme huiles utilisables dans les compositions permettant de mettre en oeuvre l'invention, on peut citer les huiles minérales, les huiles d'origine végétale (huile d'abricot, huile de tournesol, de prune), les huiles d'origine animale, les huiles de synthèse, les huiles siliconées et les huiles fluorées (perfluoropolyéthers). On peut aussi utiliser comme matières grasses des alcools gras (alcool cétylique), des acides gras, des cires (cire d'abeilles).

Comme émulsionnants et coémulsionnants utilisables dans l'invention, on peut citer par exemple les esters d'acide gras et de polyéthylène glycol tels que le stéarate de PEG-40, le stéarate de PEG-100, les esters d'acide gras et de polyol tels que le stéarate de glycéryle et le tristéarate de sorbitane.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyethylenes.

Les modes d'administration, les posologies et les formes galéniques optimales des composés et compositions selon l'invention peuvent être déterminés selon les critères généralement pris en compte dans l'établissement d'un traitement cosmétique, de préférence dermatologique, adapté à un patient comme par exemple le poids corporel du patient, l'excès de graisse constaté, l'aspect du tissu cellulitique, la tolérance au traitement, le type de peau.

La composition selon l'invention peut contenir d'autres actifs, tels que les peptides du soja, les tripeptides constitués des acides aminés Glycine, Histidine et Lysine, les mélanges de ces peptides et tripeptides, et/ou au moins un alpha-hydoxy acide du type acide lactique, notamment pour traiter et/ou prévenir les vergetures, ou encore lorsque la composition est utilisée comme agent anti-relâchement de la peau et/ou des muqueuses, ou pour résister au stress sous une contrainte physique telle qu'une prise de poids ou une distension des tissus conjonctifs en particulier dans le cadre d'une grossesse.

Dans un mode de réalisation particulier selon l'invention, la composition cosmétique ou pharmaceutique contient entre 0,1 et 10 % en poids d'un extrait riche en lupéol, avantageusement entre 0,1 et 5 % en poids, en particulier entre 1 et 5 % en poids, par rapport au poids total de la composition, ainsi que des peptides du soja du type protéines hydrolysées du soja, ou des tripeptides constitués des acides aminés Glycine, Histidine et Lysine. Les peptides du soja ou les tripeptides sont présents dans la composition à une teneur avantageusement comprise entre 0,1 et 10 % en poids, encore plus avantageusement entre 1 et 5 % en poids, encore plus avantageusement entre 2 et 4 % en poids, par rapport au poids total de la composition. Avantageusement, la composition contient en outre un alpha-hydoxy acide, tel que l'acide lactique. L'alpha-hydoxy acide est présent dans la composition à une teneur avantageusement comprise entre 0,1 et 20 % en poids, encore plus avantageusement entre 10 et 15 % en poids, par rapport au poids total de la composition.

Les peptides du soja qui sont avantageusement ajoutés dans la composition dans le cadre de la présente invention peuvent être tout peptide obtenu par hydrolyse de protéines extraites du soja, selon des conditions opératoires connues de l'homme du métier, en d'autres termes tout hydrolysat de protéine du soja. De préférence, ces peptides du soja sont des peptides ayant subi en outre une fermentation par une souche de micro-organisme. D'une manière générale, on obtient un peptide du soja fermenté en plaçant un peptide du soja dans un fermenteur en présence de glucose, de sels minéraux et d'une souche de micro-organisme donnée, dans des conditions contrôlées de température, de pH, d'oxygénation et de durée. Après la fermentation, on obtient le peptide du soja fermenté par des opérations de séparation et de filtration classiques. Cette technique est notamment mise en oeuvre par la société COLETICA qui commercialise ainsi divers hydrolysats de protéines végétales fermentés. De préférence, les peptides du soja, fermentés ou non, dans la composition utilisée selon la présente invention, ont un poids moléculaire compris entre environ 200 et environ 20000 Daltons, tel que mesuré par exemple par électrophorèse.

Un peptide du soja particulièrement préféré pour la composition utilisée selon l'invention est le peptide fermenté dénommé « Phytokine^{®}», tel que commercialisé par la société COLETICA.

Ce peptide du soja fermenté spécifique, de poids moléculaire moyen d'environ 800 Daltons, est obtenu par fermentation d'un peptide du soja par la souche de micro-organisme Lactobaccillus et son aminogramme est le suivant :

| | Nombre de résidus pour 100 |
|---|---|
| Hyp .................................................................... | 0,39 |
| Asp .................................................................... | 12,64 |
| Thr ..................................................................... | 2,93 |
| Ser ..................................................................... | 4,29 |
| Glu ..................................................................... | 20,08 |
| Pro ..................................................................... | 7,31 |
| Gly ..................................................................... | 7,95 |
| Ala ..................................................................... | 7,76 |
| Cys ..................................................................... | ND* |
| Val ..................................................................... | 5,59 |
| Met .................................................................... | 0,96 |
| Ile ....................................................................... | 4,46 |
| Leu..................................................................... | 7,42 |
| Tyr..................................................................... | 1,38 |
| Phe ..................................................................... | 3,39 |
| His ..................................................................... | 2,12 |
| Hyl ..................................................................... | 0,09 |
| Lys ..................................................................... | 5,73 |
| Trp ..................................................................... | ND* |
| Arg .................................................................. | 5,53 |
| ßAla ................................................................... | ND |

| | |
|---|---|
| (*ND : non déterminé) | |

Par « tripeptides constitués des acides aminés Glycine, Histidine et Lysine », on entend en particulier les tripeptides de séquence Gly-His-Lys, dont les acides aminés peuvent être sous la forme D, L ou DL, éventuellement conjugués avec un acide carboxylique tel que l'acide acétique, sous forme d'un complexe avec un métal tel que le zinc ou le cuivre.

Parmi les tripeptides constitués des acides aminés Glycine, Histidine et Lysine, on préfère utiliser le tripeptide « KOLLAREN-CPP », dont la dénomination INCI est « tripeptide-1 », tel que commercialisé par la société SEPORGA. Le « KOLLAREN-CPP » est un tripeptide de séquence Gly-His-Lys conjuguée avec l'acide acétique (acétate), sous forme d'un complexe avec du zinc.

L'α-hydroxy acide peut être tout α-hydroxy acide permettant d'obtenir un effet d'exfoliation et/ou d'hydratation de la peau, tels que, par exemple, l'acide citrique, l'acide pyruvique, l'acide glycolique ou encore l'acide lactique.

Un α-hydroxy acide particulièrement préféré pour la composition utilisée selon l'invention est l'acide lactique.

La composition utilisée selon l'invention comprend avantageusement en outre un composé destiné à régler le pH de la composition selon l'invention, à une valeur comprise entre environ 2 et environ 4, et de préférence une valeur d'environ 3,5, notamment pour neutraliser partiellement l'α-hydroxy acide. En particulier, on peut utiliser l'arginine ou une alcanolamine, telle que la triéthanolamine.

Dans un mode de réalisation particulier de la présente invention, le lupéol est obtenu à partir de coques de lupin avantageusement choisi dans le groupe constitué par le lupinus angustifolius, le lupinus albus, le lupinus luteus, le lupinus mutabilis, le lupinus graecus, le lupinus micranthus Guss, le lupinus hispanicus, le lupinus pilosus, le lupinus cosentinii, le lupinus atlanticus, le lupinus princei et le lupinus somaliensis. Selon une variante avantageuse de l'invention, le lupéol est obtenu à partir de coques de lupinus albus, de préférence le lupinus albus de variété très portant le gène pauper.

Les exemples suivants illustrent la présente invention.

### EXEMPLE 1 : Composition d'un extrait riche en lupéol

Le tableau 1 suivant donne une composition particulière d'extrait riche en lupéol utilisé dans le cadre de la présente invention, ainsi que les spécifications que doit satisfaire un extrait de lupéol pour pouvoir être considéré comme étant un extrait riche en lupéol selon la présente invention.

**Tableau 1**

| **Critère de composition** | **Résultats** | **Spécifications** |
|---|---|---|
| Aspect | Solide beige à jaune clair | Solide beige à jaune clair |
| Teneur en lupéol (g/100g) | 84,7 | 75,0-90,0 |
| Teneur en stérols totaux (g/100g) | 12,2 | 8,0-20,0 |
| Composition relative en stérols (%) | | |
| Béta-sitostérol | 72,9 | 65,0-80,0 |
| Campestérol | 5,7 | 2,0-10,0 |
| Stigmastérol | 21,4 | 15,0-30,0 |
| Perte à la dessication (g/100g) (1) | 0,03 | < 0,2 |

| | | |
|---|---|---|
| (1) 1 heure à 105°C | | |

### EXEMPLE 2: Effets du lupéol sur l'expression du gène codant pour la protéine de stress HSP47.

Nous avons utilisé la méthode des « cDNA micro array » pour étudier les effets du produit sur l'expression des gènes codant pour des protéines structurales et régulatrices d'intérêt potentiel dans la physiologie cutanée. Une telle approche permet de « screener » en une seule étape les effets d'un produit ou d'un traitement sur l'expression des gènes dans un système biologique donné et d'avoir une signature des effets de ce traitement.

### Conditions de culture et produits à l'essai

Le produit a été appliqué sur des fibroblastes cutanés humains cultivés dans du MEM/M199 pendant 18 heures. Le lupéol, en solution dans l'éthanol a été testé à la concentration de 4,5 µg/ml, cette concentration correspond à une dose non cytotoxique.

### Analyse de l'expression différentielle des gènes

La méthodologie utilisée est celle préconisée par Clontech (Palo Alto, USA), et comprend :
- une étape d'extraction et de purification des ARN totaux ;
- une étape de purification des ARN messagers selon le protocole AtlasPure (Clontech) ;
- le marquage des sondes ADN au P³² par reverse-transcription ;
- la purification des sondes marquées par chromatographie sur colonne d'exclusion et vérification de la qualité et de l'équivalence par comptage en scintillation liquide ; et
- hybridation des membranes (fournies par la société Custom ATLAS BIOAlternative) avec les sondes radio-marquées (68°C, une nuit).

### Analyse des résultats

L'analyse des membranes a lieu par quantification directe de la radioactivité des spots à l'aide d'un PhosphoImager Cyclone (fourni par la société Packard Instrument).

Les résultats sont exprimés en unités relatives (UR, radioactivité moyenne des points du double spot correspondant à chaque gène, corrigé du bruit de fond et des différences d'intensité de marquage des sondes). Dans cette expérience, on a considéré de façon arbitraire qu'un gène était exprimé de façon significative lorsque son UR était supérieur ou égale à 2.

Les résultats ont été analysés au cas par cas, marqueur par marqueur. Arbitrairement, la limite de significativité a été fixée à environ +30% du contrôle pour un effet stimulant, c'est-à-dire pour un effet supérieur à 130%.

### Résultats

Dans nos conditions expérimentales, une incubation de 18 heures en présence de 4,5 µg/ml de lupéol induit une régulation positive de l'expression du gène codant pour l'HSP47 (cf tableau 2).

**Tableau 2**

| | Cellules contrôles | Cellules + Lupéol 4,5 µg/ml, 18 heures |
|---|---|---|
| UR | 28,2 | 36,4 (129% du contrôle) |

### Conclusion

Les résultats de l'exemple 2 suggère que le lupéol pourrait de manière tout à fait inattendue stimuler le gène codant pour la protéine de stress HSP47 dans des fibroblastes cutanés humains en culture.

### EXEMPLE 3: Effets du lupéol sur l'expression de la protéine de stress HSP47 dans un modèle de fibroblastes en culture

Afin de vérifier cette stimulation potentielle de l'HSP47 révélée par le screening, nous avons étudié l'expression de la protéine HSP47 par la technique du westem-blot. Cette technique consiste en :
- une séparation des différentes protéines sur un gel de polyacrylamide en conditions dénaturantes ;
- un transfert des protéines sur une membrane spécifique (membrane PVDF, commercialisée par la société Biorad) ;
- un Marquage de la protéine d'intérêt par un anti-corps spécifique (HSP47, Stressgen) ;
- une Révélation « colorimétrique » (Sigma Fast, BCPIP/NBT).

Différentes concentrations non cytotoxiques (5, 10 et 20 µg/ml), ainsi que plusieurs temps d'incubation (24 et 48 heures) ont été testés.

Dans nos conditions expérimentales, nous avons enregistré une stimulation de la production d'HSP47 pour une dose de 20 µg/ml et un temps d'incubation de 48h.

Les inventeurs ont montré, de manière tout à fait surprenante, en utilisant la technique des micro arrays, ainsi que la technique du western-blot, que le lupéol stimulait la production de l'HSP47 au niveau transcriptionnel (ARNm) et traductionnel, c'est-à-dire au niveau des protéines.

### EXEMPLE 4: Effets du lupéol sur des fibroblastes sains et des fibroblastes de vergetures

### a) Cellules

Les fibroblastes sains (FS), les fibroblastes de vergeture rouge ou récente (FVr), et les fibroblastes de vergeture blanche plus ancienne (FVb) ont été utilisés dans cette étude.

### b) Traitement

Les fibroblastes ont été cultivés en l'absence ou en présence de lupéol à la concentration de 7 µg/ml.

### c) Effet du lupéol sur les capacités de rétraction des fibroblastes cultivés dans un lattice libre

### Principe

Le mécanisme de la rétraction consiste en un rassemblement et réarrangement du collagène par les fibroblastes. Le collagène se polymérise en fibrilles sur lesquelles s'attachent les cellules. Ces dernières tirent sur les fibrilles grâce à leur mouvement migratoire, et réorganisent ainsi la matrice. Le lattice voit alors son diamètre diminuer.

### Méthode

Le mélange de fabrication des lattices a été coulé dans des boîtes de Pétri de 50 mm de diamètre. En quelques minutes à 37°C, un gel s'est constitué. Les lattices ont été cultivés avec ou sans lupéol. Le taux de rétraction a été déterminé par la mesure des diamètres des lattices.

### Résultats

Dans les premiers jours, le diamètre du gel diminue fortement. Les FS rétractent plus vite la matrice que les FVr et les FVb. Aucune différence significative n'est visible entre les FVr et les FVb.

Les résultats sont présentés dans le tableau 3 suivant. Par exemple, à J1 : les lattices sont rétractés à 42% de leur diamètre initial pour FS contre 29% pour FV. L'ajout du lupéol ralentit la rétraction. Par exemple, à J2 : le lupéol ralentit la rétraction de 20% pour les FS, 20% pour les FVr et 10% pour les FVb.

**Tableau 3**

| | FS | FS + Lupéol | FVr | FVr + lupéol | FVb | FVb + lupéol |
|---|---|---|---|---|---|---|
| Diamètre du gel | 2,5 | 3 | 3 | 3,6 | 2,9 | 3,2 |
| **% de diminution de la rétraction** | - | **20** | **-** | **20** | - | **10** |

Le lupéol diminue donc les capacités de rétraction des fibroblastes cultivés dans un lattice de collagène.

### d) Effet du lupéol sur les forces isométriques développées par des fibroblastes cultivés dans un lattice libre

### Principe

L'inhibition mécanique de la rétraction du gel de collagène dans lequel les fibroblastes sont inclus se traduit par la génération d'une force, appelée "force de rétraction" ou "force isométrique".

Les lattices se développent dans une boîte de culture qui est constituée de 8 cuves rectangulaires. Dans chacune plongent 2 lames flexibles en silicium, dont les parties inférieures sont constituées de grilles sur lesquelles s'accrochent le lattice lors de sa polymérisation. Le lattice se développe entre 2 lames pour aboutir à une forme rectangulaire légèrement rétrécie au centre. Cette forme en mécanique classique est désignée comme une forme en "diabolo ". Ces lames sont équipées, au niveau de leur partie supérieure, d'un système de jauge de contrainte en or déposé à leur surface. Sous l'influence de la force de rétraction développée par les fibroblastes, les lames de silicium se déforment. Cela se traduit par une variation de la valeur de résistance électrique de la jauge de contrainte, mesurée par l'intermédiaire d'un pont de Wheastone. Cette variation indique la force développée au sein du lattice, mesurée en temps réel par le biais d'une carte d'acquisition PC et d'un logiciel adapté.

### Méthode

Le mélange de fabrication des lattices a été coulé dans les cuves rectangulaires de la boîte de culture. En quelques minutes à 37°C, un gel s'est constitué. Le milieu de culture DMEMc avec (7 µg/ml) ou sans lupéol a été ajouté. Les forces isométriques ont été mesurées durant 48 heures.

### Résultats

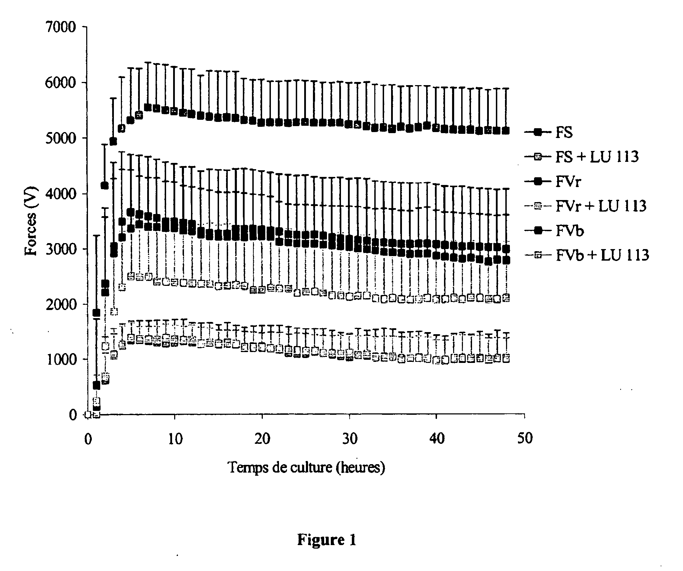

La Figure 1 présente les résultats de mesure des forces isométriques en fonction du temps de culture. Les forces isométriques augmentent pendant les 8 premières heures de culture. Aucune différence significative n'est observée entre les FS et les FVb. Après 7 heures de culture, les FVr développent des forces isométriques significativement plus importantes que celles des FS et FVb (FVr : 5547 V ± 808 ; FS : 3583 V ± 1076 ; FVb : 3999 V ± 881). Pour chacune des 3 lignées cellulaires, l'ajout du lupéol diminue significativement les forces isométriques (à 7 heures de culture : FVr + LU 113 : 2488 V ± 1065 ; FS + LU 113 : 1325 V ± 372 ; FVb + LU 113 : 1365 V ± 228).

Ainsi, le lupéol diminue les forces isométriques des fibroblastes sains et de vergetures (rouges et blanches) cultivés en lattice tendu.

### e) Effet du lupéol sur la morphologie des fibroblastes cultivés en lattice libre

### Principe

Les fibroblastes sont des cellules de grande taille (100 µm) avec une morphologie fusiforme ou étoilée. Leur cytoplasme contient un ensemble de filaments protéiques (filaments intermédiaires, microtubules, filaments d'actine) qui en constituent l'ossature, ou cytosquelette, en étroite relation avec la membrane plasmique. 10% de toutes les protéines cellulaires sont constituées d'actine, polymérisée sous forme de filaments et de faisceaux, qui sont souvent décrits sous le nom de *stress fibers.* Les filaments d'actine participent à l'activité contractile du fibroblaste en générant les forces isométriques. Il existe plusieurs types d'actine, dont l'actine fibrillaire polymérisée (F actine) et l'α actine de muscle lisse ou α smooth muscle actine (αSM actine).

### Méthode

### 1. Mise en évidence de la F actine

Les lattices tendus sur anneau de nylon ont été cultivés pendant 5 jours dans des boites de Pétri (Ø 50 mm). Ils ont été fixés avec du paraformaldéhyde à 3% dans PBS pendant 20 min, puis rincés avec du PBS. Ils ont ensuite été incubés avec PBS + 0.1 %Triton X100 pour perméabiliser la membrane. Les lattices ont été incubés avec la phalloidine-FITC (2.5 µg/ml) pendant 30 min à l'obscurité, et rincés avec du PBS. Ils ont été montés entre lame et lamelle avec une goutte de Vectashield, puis ont été observés au microscope à fluorescence sous UV.

### 2. Mise en évidence de l'αSM actine

Les lattices tendus sur anneau de nylon ont été cultivés pendant 5 jours dans des boites de Pétri (Ø 50 mm). Ils ont été fixés avec du paraformaldéhyde à 3% dans PBS pendant 20 min, puis rincés avec du PBS. Ils ont ensuite été incubés avec PBS + 0.1%Triton X100 pour perméabiliser la membrane aux anticorps, puis avec H₂O₂ 3% pour éliminer les peroxydases endogènes. Les liaisons aspécifiques ont été bloquées avec BSA 3% + 10% sérum de mouton dans PBS. Les lattices ont ensuite été incubés, pendant une nuit avec l'anticorps primaire de souris anti-αSM actine dilué au 1/100, puis rincés avec PBS + BSA. L'anticorps secondaire anti-IgG de souris couplé à la péroxidase dilué au 1/150 a été appliqué pendant une heure puis rinçé avec PBS + BSA. La révélation de la localisation du marquage a été réalisée avec une solution de DAB qui provoque un noircissement de la peroxydase. Les lattices ont été contre-colorés à l'hématoxyline de Harris pendant 1-2 min. Ils ont été montés entre lame et lamelle, puis ont été observés au microscope optique.

### Résultats

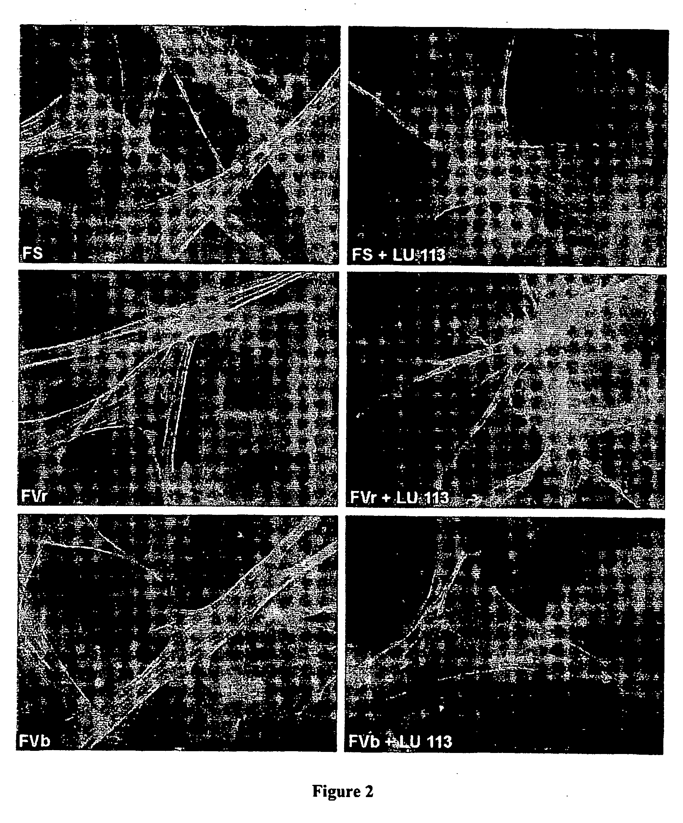

### 1. Mise en évidence de la F actine (Cf. Figure 2)

Les fibroblastes FS, FVr et FVb sont allongés et fusiformes. Leur cytoplasme possède des fibres de stress de F actine.

Ainsi, en présence du lupéol, les fibroblastes FS, FVr et FVb perdent leurs fibres de stress de F actine et évoluent vers une forme étoilée.

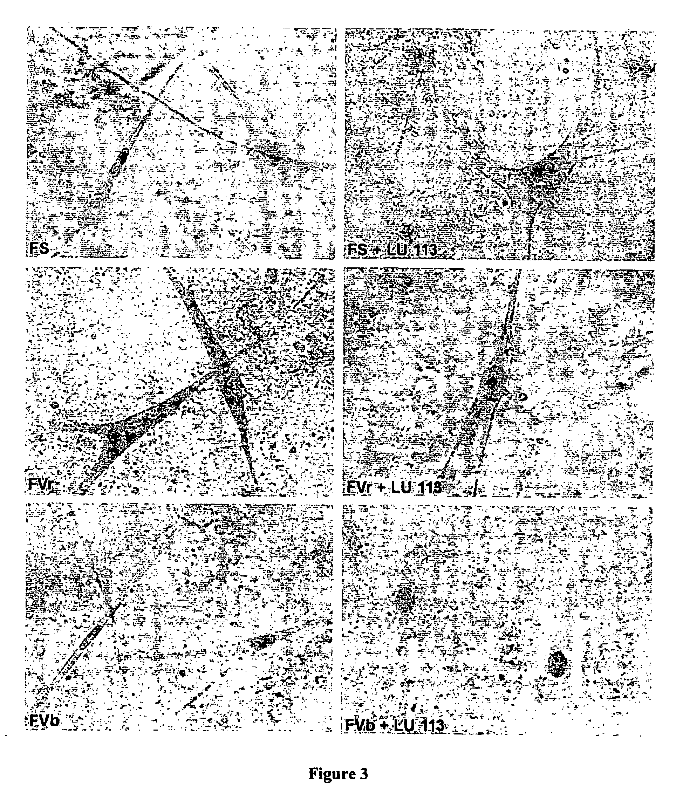

### 2. Mise en évidence de l'aSMactine (Cf. Figure 3)

L'αSM actine, révélée par la coloration marron, est présente sous forme granulaire dans le cytoplasme des fibroblastes FVr. Les fibroblastes FS et FVb contiennent peu d'αSM actine sous forme granulaire.

En présence du lupéol, les fibroblastes FS, FVr et FVb montrent un faible marquage de l'αSM actine.

Ainsi, en présence du lupéol (principe actif LU 113), la répartition cytoplasmique du réseau d'actine n'est pas conservée.

### f) Conclusion

En conclusion, il a ici été montré que le lupéol modifiait :
- les propriétés mécaniques des fibroblastes sains et de vergetures : capacités de rétraction et forces isométriques,
- la morphologie des fibroblastes, avec une absence des fibres de stress.

Les vergetures apparaissent aux endroits où la peau est soumise à des tensions excessives et en cas de fragilisation du derme, soit de façon constitutionnelle, soit par troubles métaboliques, hormonaux ou autres. Les fibroblastes de vergeture rouge (vergetures récentes et évolutives) ont une capacité contractile importante, à savoir des forces isométriques élevées et un réseau de fibres de stress bien développé.

Cette étude montre que le lupéol favorise la décontraction cutanée (diminution des forces isométriques et des fibres d'actine), et que son action pourrait donc être bénéfique au sein des vergetures.

Dans les exemples 5 à 10 suivants, les proportions sont données en pourcentage (p/p) et les abréviations QS signifient Quantité Suffisante, QSP signifient Quantité Suffisante Pour.

### EXEMPLE 5 : Bain de bouche

| **Extrait riche en lupéol** | **0,1 à 10%** |
|---|---|
| Alcool éthylique | 10% |
| Glycérine Huile de ricin hydrogénée, | 10% |
| Ethoxylée à 40 moles EO (Crémophor co410) | 0,5% |
| Poly(Methyl vinyl ether/Acide Maléïque (Gantrez S97BF) | 0,2% |
| Soude | 0,15% |
| Fluorure de sodium | 0,05% |
| Arôme Cannelle-Menthe | 0,1% |
| Triclosan | 0,03% |
| Chlorure de zinc | 0,01 % |
| Saccharine sodique | 0,01 % |
| Colorant C.I. 16255 (E 124) | 0,0025% |
| Eau purifiée | QSP 100% |

### EXEMPLE 6 : Pâte dentifrice

| **Extrait riche en lupéol** | **0,1 à 10%** |
|---|---|
| Monofuorophosphate de sodium | 0,75% |
| Fluorure de Sodium | 0,10% |
| Sorbitol à 70% | 35% |
| Silice synthétique à fort pouvoir abrasif | 13% |
| Silice synthétique à faible pouvoir abrasif | 5% |
| Carboxymethylcellulose sodique | 1,6% |
| Lauryl sulfate de sodium | 1% |
| Arôme mentholé | 0,85% |
| Oxyde de titane | 0,5% |
| Lessive de soude | 0,5% |
| Cyclamate de sodium | 0,3% |
| Menthol | 0,15% |
| Saccharine sodique | 0,07% |
| Eau purifiée | QSP 100% |

### EXEMPLE 7 : Crème anti-vergeture

| Eau | QSP 100% |
|---|---|
| Acide Lactique | 10,0% |
| Triéthanolamine | 7,5% |
| Cyclométhicone | 5,4% |
| Ethylhéxyle Palmitate | 4,5% |
| Glycérides de Coco Hydrogenées | 3,0% |
| Méthylsilanol de Lactate de Sodium | 3,0% |
| Propylène Glycol | 2,5% |
| Néopentanoate Isodécylique | 2,0% |
| Protéine Hydrolysée de Soja | 2,0% |
| Stéarate de Glycérol | 1,7% |
| Alcool Arachidique | 1,6% |
| Alcool Cétylique | 1,3% |
| Acide Stéarique | 1,0% |

| **Extrait riche en lupéol** | **0,1 à 10%** |
|---|---|
| Alcool Béhénylique | 0,9% |
| Polyacrylamide | 0,8% |
| Extrait de feuilles de Sophora Japonica | 0,5% |
| Glucoside Arachidique | 0,4% |
| Cire d'abeille | 0,4% |
| Isoparaffine C13-14 | 0,4% |
| DEA-Phosphate Cétylique | 0,3% |
| Gluconate de Zinc | 0,2% |
| Glycérine | 0,1 % |
| Alcool Cétéarylique | 0,1% |
| Palmitate Cétylique | 0,1% |
| Glycérides de Coco | 0,1 % |
| Laureth-7 | 0,1% |
| Extrait d'Enteromorpha Compressa | 0,1 % |
| Parfum | QS |
| Conservateurs | QS |

### EXEMPLE 8 : Crème anti-vergeture

| Eau | QSP 100% |
|---|---|
| Acide Lactique | 10,0% |
| Triéthanolamine | 7,5% |
| Cyclométhicone | 5,4% |
| Ethylhéxyle Palmitate | 4,5% |
| Glycérides de Coco Hydrogenées | 3,0% |
| Méthylsilanol de Lactate de Sodium | 3,0% |
| Propylène Glycol | 2,5% |
| Néopentanoate Isodécylique | 2,0% |
| Tripeptides de séquence Gly-His-Lys | 2,0% |
| Stéarate de Glycérol | 1,7% |
| Alcool Arachidique | 1,6% |
| Alcool Cétylique | 1,3% |
| Acide Stéarique | 1,0% |

| **Extrait riche en lupéol** | **0,1 à 10%** |
|---|---|
| Alcool Béhénylique | 0,9% |
| Polyacrylamide | 0,8% |
| Extrait de feuilles de Sophora Japonica | 0,5% |
| Glucoside Arachidique | 0,4% |
| Cire d'abeille | 0,4% |
| Isoparaffine C13-14 | 0,4% |
| DEA-Phosphate Cétylique | 0,3% |
| Gluconate de Zinc | 0,2% |
| Glycérine | 0,1% |
| Alcool Cétéarylique | 0,1% |
| Palmitate Cétylique | 0,1% |
| Glycérides de Coco | 0,1% |
| Laureth-7 | 0,1% |
| Extrait d'Enteromorpha Compressa | 0,1% |
| Parfum | QS |
| Conservateurs | QS |

### EXEMPLE 9 : Crème anti-vergeture

| Eau | QSP 100% |
|---|---|
| Acide Lactique | 10,0% |
| Triéthanolamine | 7,5% |
| Cyclométhicone | 5,4% |
| Ethylhéxyle Palmitate | 4,5% |
| Glycérides de Coco Hydrogenées | 3,0% |
| Méthylsilanol de Lactate de Sodium | 3,0% |
| Propylène Glycol | 2,5% |
| Néopentanoate Isodécylique | 2,0% |
| Protéine Hydrolysée de Soja | 1,0% |
| Tripeptides de séquence Gly-His-Lys | 2,5% |
| Stéarate de Glycérol | 1,7% |
| Alcool Arachidique | 1,6% |
| Alcool Cétylique | 1,3% |
| Acide Stéarique | 1,0% |

| **Extrait riche en lupéol** | **0,1** à **10%** |
|---|---|
| Alcool Béhénylique | 0,9% |
| Polyacrylamide | 0,8% |
| Extrait de feuilles de Sophora Japonica | 0,5% |
| Glucoside Arachidique | 0,4% |
| Cire d'abeille | 0,4% |
| Isoparaffine C13-14 | 0,4% |
| DEA-Phosphate Cétylique | 0,3% |
| Gluconate de Zinc | 0,2% |
| Glycérine | 0,1% |
| Alcool Cétéarylique | 0,1% |
| Palmitate Cétylique | 0,1% |
| Glycérides de Coco | 0,1% |
| Laureth-7 | 0,1% |
| Extrait d'Enteromorpha Compressa | 0,1% |
| Parfum | QS |
| Conservateurs | QS |

### EXEMPLE 10 : Gel restructurant

| **Eau** | **QSP 100%** |
|---|---|
| PEG-6 | 3,60% |
| Butylène glycol | 2,70% |
| Dextrine | 1,86% |
| Triméthicone de Phényl | 1,20% |

| **Extrait riche en lupéol** | **0,1 à 10%** |
|---|---|
| crosspolymère Acrylates/ acrylate d'alkyl C10-30 | 0,60% |
| Crosspolymère Diméthicone/ Diméthicone de Phényle vinyle | 0,30% |
| Extrait de Fleurs de Sophora Japonica | 0,20% |
| Gomme Xanthane | 0,15% |
| PPG 26-Buteth-26 | 0,11% |
| 4,5,7-Trihydroxyisoflavone | 0,10% |
| Protéine de Soja Hydrolysée | 0,10% |
| Glucose | 0,08% |
| Huile de ricin hydrogénée PEG 40 | 0,07% |
| Sorbitol | 0,04% |
| Extrait de Centella Asiatica | 0,04% |
| Glycérine | 0,04% |
| Acide Citrique | 0,02% |
| Extrait d'Enteromorpha Compressa | 0,02% |
| Parfum | QS |
| Conservateurs | QS |

## Revendications

1. Composition pharmaceutique ou cosmétique comprenant un extrait riche en lupéol pour son utilisation dans le traitement ou la prévention d'une dégénérescence des tissus conjonctifs, au niveau du derme et/ou du cartilage, notamment du cartilage articulaire, et/ou du tissu conjonctif gingival, liée à une anomalie de la production de collagène, en particulier du collagène du type I, II, III, IV ou V.

2. Composition pour son utilisation selon la revendication 1 pour activer la synthèse des protéines de stress, notamment de l'HSP47.

3. Composition pour son utilisation selon l'une quelconque des revendications précédentes, **caractérisée en ce que** la composition pharmaceutique est destinée à traiter et/ou prévenir les pathologies articulaires, telles que l'arthrose, les maladies parodontales, telles que la gingivite ou la parodontite, à traiter les vergetures ou à prévenir leur apparition.

4. Composition pour son utilisation selon l'une quelconque des revendications 1 à 2. en tant qu'agent cicatrisant, agent restructurant et agent anti-relâchement de la peau et/ou des muqueuses.

5. Composition pour son utilisation selon la revendication 4, **caractérisée en ce que** la composition cosmétique permet aux tissus conjonctifs de résister au stress sous une contrainte physique telle qu'une prise de poids, une surcharge pondérale, une distension desdits tissus notamment dans le cadre d'une grossesse, une irradiation, notamment aux ultra-violets.

6. Composition pour son utilisation selon la revendication 4 ou 5, pour prévenir et/ou retarder le vieillissement cutané, pour prévenir et/ou traiter les vergetures ou leur apparition.

7. Composition pour son utilisation selon l'une quelconque des revendications 4 à 6, **caractérisée en ce que** la composition cosmétique est destinée aux femmes enceintes.

8. Composition pour son utilisation selon la revendication 4, en tant que bain de bouche ou pâte dentifrice.

9. Composition pour son utilisation selon l'une quelconque des revendications 4 à 8, caractériséee en ce que la composition cosmétique est à application topique.

10. Composition pour son utilisation selon l'une quelconque des revendications 4 à 9, **caractérisée en ce que** la composition cosmétique comprend entre 0,001 et 10% en poids d'extrait riche en lupéol.

11. Composition pour son utilisation selon l'une quelconque des revendications précédentes, **caractérisé en ce que** l'extrait présente un taux de lupéol supérieur à 30% en poids, de préférence supérieur à 50% en poids.

12. Composition pour son utilisation selon la revendication 11, **caractérisé en ce que** l'extrait présente un taux de lupéol compris entre 70 et 100% en poids.

13. Composition pour son utilisation selon l'une quelconque des revendications précédentes, **caractérisée en ce que** le lupéol est obtenu à partir des coques de lupinus albus, de préférence le lupinus albus de variété Arès portant le gène pauper.

## Claims

1. Pharmaceutical or cosmetic composition comprising a lupeol-rich extract for the use thereof for treating or preventing connective tissue degeneration in the dermis and/or the cartilage, in particular the articular cartilage, and/or the gingival connective tissue, related to a collagen, in particular collagen type I, II, III, IV or V, production anomaly.

2. Composition for the use thereof according to claim 1 for activating the synthesis of heat shock proteins, in particular of HSP47.

3. Composition for the use thereof according to either one of the preceding claims, **characterised in that** the pharmaceutical composition is for treating and/or preventing articular pathologies such as arthrosis, or periodontal diseases such as gingivitis or periodontitis, for treating stretch marks or for preventing the appearance thereof.

4. Composition for the use thereof according to either one of claims 1 and 2 as a healing agent, a restructuring agent and an anti-sagging agent for the skin and/or the mucous membranes.

5. Composition for the use thereof according to claim 4, **characterised in that** the cosmetic composition allows the connective tissues to withstand shock(s) under physical stress, such as weight gain, excess weight, or distension of said tissues in particular in the context of a pregnancy, or irradiation, in particular ultraviolet irradiation.

6. Composition for the use thereof according to claim 4 or 5 for preventing and/or delaying skin aging, and/or for preventing and/or treating stretch marks or the appearance thereof.

7. Composition for the use thereof according to any one of claims 4 to 6, **characterised in that** the cosmetic composition is intended for pregnant women.

8. Composition for the use thereof according to claim 4 as a mouthwash or toothpaste.

9. Composition for the use thereof according to any one of claims 4 to 8, **characterised in that** the cosmetic composition is for topical application.

10. Composition for the use thereof according to any one of claims 4 to 9, **characterised in that** the cosmetic composition comprises between 0.001 and 10% by weight of lupeol-rich extract.

11. Composition for the use thereof according to any one of the preceding claims, **characterised in that** the extract has a lupeol content of greater than 30% by weight, preferably greater than 50% by weight.

12. Composition for the use thereof according to claim 11, **characterised in that** the extract has a lupeol content of between 70 and 100% by weight.

13. Composition for the use thereof according to any one of the preceding claims, **characterised in that** the lupeol is obtained from pods of lupinus albus, preferably the lupinus albus variety Ares carrying the pauper gene.

## Patentansprüche

1. Pharmazeutische oder kosmetische Zusammensetzung mit einem lupeolreichen Extrakt zu deren Verwendung bei der Behandlung oder Prävention einer Degeneration des Bindegewebes, im Bereich der Dermis und/oder der Knorpel, insbesondere der Gelenksknorpel, und/oder des Zahnfleischbindegewebes, die mit einer Anomalie bei der Produktion von Kollagen verbunden ist, insbesondere von Kollagen des Typs I, II, III, IV oder V.

2. Zusammensetzung zu deren Verwendung nach Anspruch 1 zum Aktivieren der Synthese von Hitzeschockproteinen, insbesondere von HSP47.

3. Zusammensetzung zu deren Verwendung nach einem der vorangehenden Ansprüche, **dadurch gekennzeichnet, dass** die pharmazeutische Zusammensetzung für die Behandlung und/oder Prävention von Gelenkpathologien, wie etwa Arthrose, Parodontalerkrankungen, wie etwa Zahnfleischentzündung oder Parodontitis, für die Behandlung von Schwangerschaftstreifen oder zur Prävention von deren Erscheinen bestimmt ist.

4. Zusammensetzung zu deren Verwendung nach einem der Ansprüche 1 bis 2 als Narbenbildungsmittel, Restrukturierungsmittel und als Mittel zum Schutz vor Erschlaffung der Haut und/oder der Schleimhäute.

5. Zusammensetzung zu deren Verwendung nach Anspruch 4, **dadurch gekennzeichnet, dass** die kosmetische Zusammensetzung es dem Bindegewebe ermöglicht, dem Stress bei einer physischen Belastung, wie etwa bei einer Gewichtszunahme, bei Übergewicht, bei einer Ausdehnung des Gewebes insbesondere im Rahmen einer Schwangerschaft, einer Bestrahlung, insbesondere einer UV-Bestrahlung standzuhalten.

6. Zusammensetzung zu deren Verwendung nach Anspruch 4 oder 5 zur Prävention und/oder Verzögerung der Hautalterung, zur Prävention und/oder Behandlung von Schwangerschaftsstreifen oder deren Erscheinen.

7. Zusammensetzung zu deren Verwendung nach einem der Ansprüche 4 bis 6, **dadurch gekennzeichnet, dass** die kosmetische Zusammensetzung für schwangere Frauen bestimmt ist.

8. Zusammensetzung zu deren Verwendung nach Anspruch 4 als Mundbad oder Zahnpasta.

9. Zusammensetzung zu deren Verwendung nach einem der Ansprüche 4 bis 8, **dadurch gekennzeichnet, dass** die kosmetische Zusammensetzung zur lokalen Anwendung vorgesehen ist.

10. Zusammensetzung zu deren Verwendung nach einem der Ansprüche 4 bis 9, **dadurch gekennzeichnet, dass** die kosmetische Zusammensetzung zwischen 0,001 und 10 Gew.-% an lupeolreichen Extrakt enthält.

11. Zusammensetzung zu deren Verwendung nach einem der vorangehenden Ansprüche, **dadurch gekennzeichnet, dass** das Extrakt einen Lupeolgehalt von über 30 Gew.-%, vorzugsweise von über 50 Gew.-% enthält.

12. Zusammensetzung zu deren Verwendung nach Anspruch 11, **dadurch gekennzeichnet, dass** das Extrakt einen Lupeolgehalt von zwischen 70 und 100 Gew.-% aufweist.

13. Zusammensetzung zu deren Verwendung nach einem der vorangehenden Ansprüche, **dadurch gekennzeichnet, dass** das Lupeol ausgehend von Schalen der weißen Lupine, vorzugsweise der weißen Lupine der Sorte Ares mit dem Gen "Pauper" gewonnen wird.
